# EUROPEAN PATENT SPECIFICATION

(11) **EP 4 313 106 B1**
(45) Date of publication and mention of the grant of the patent: **15.01.2025**
(21) Application number: 22717817.5
(22) Date of filing: 25.03.2022
(51) Int. Cl.: A61K 38/095, A61P 1/06

(54) **TREATMENT OF DYSPHAGIA**
BEHANDLUNG VON DYSPHAGIE
TRAITEMENT DE LA DYSPHAGIE

(30) Priority: 26.03.2021 EP 21305385
(43) Date of publication of application: 07.02.2024
(73) Proprietor: OT4B, 31000 Toulouse (FR); Centre Hospitalier Universitaire De Toulouse, 31059 Toulouse Cedex 9 (FR); UNIVERSITE TOULOUSE III - PAUL SABATIER, 31062 Toulouse Cedex 9 (FR)
(72) Inventor: TAUBER, Marie-Thérèse, 31000 Toulouse (FR); VALETTE, Marion, 46230 Bach (FR); FICHAUX-BOURIN, Pascale, 31270 Cugnaux (FR); BORENSZTEIN, Pascale, 75010 Paris (FR); PETIT, Géraldine, 34830 Jacou (FR); VUILLET, François, 75017 Paris (FR)
(74) Representative: Cabinet Becker et Associés
(86) International application number: PCT/EP2022/057917
(87) International publication number: WO 2022/200568

(56) References cited:
- US-A1- 2016 128 978
- US-A1- 2020 093 884
- US-A1- 2020 093 885
- GROSS ROXANN DIEZ ET AL: "Subclinical dysphagia in persons with Prader-Willi syndrome", vol. 173, no. 2, 19 October 2016 (2016-10-19), US, pages 384 - 394, XP055831566, ISSN: 1552-4825, Retrieved from the Internet <URL:https://api.wiley.com/onlinelibrary/tdm/v1/articles/10.1002%2Fajmg.a.38015> DOI: 10.1002/ajmg.a.38015
- TAUBER MAÏTHÉ ET AL: "The Use of Oxytocin to Improve Feeding and Social Skills in Infants With Prader-Willi Syndrome", vol. 139, no. 2, 1 February 2017 (2017-02-01), US, XP055831663, ISSN: 0031-4005, Retrieved from the Internet <URL:https://pediatrics.aappublications.org/content/pediatrics/139/2/e20162976.full.pdf> DOI: 10.1542/peds.2016-2976

## Description

### Field of the invention

The invention relates to the therapeutic management of dysphagia in a subject suffering from a neurodevelopmental disorder. The invention is defined by the appended claims.

### Technological background

Swallowing in adults and children is a complex mechanism based on a coordinated operation of the mouth, pharynx and esophagus. Swallowing mechanism includes three phases, namely the oral, pharyngeal, and esophageal phases. Each phase of the swallowing process relies on key sets of muscles innervated by a subset of cranial nerves.

In the oral phase, food is brought into the mouth and chewed and mixed with saliva by jaw and tongue movements, yielding a bolus for swallowing. The pharyngeal phase begins as the bolus is pushed back on the tongue. Pharyngeal muscles pull the larynx forward, elevate the hyoid bone, and close the epiglottis, protecting the airway as the bolus traverses the pharynx. In concert, soft palate muscles elevate to close the nasopharynx, preventing aspiration into nasal sinuses. Finally, in the esophageal phase, the esophageal sphincter relaxes to allow the bolus to enter the esophagus and then closes as a peristaltic wave pushes food toward the stomach liquid.

Only the oral phase requires conscious effort. Once in the pharynx, the bolus passes into the stomach by involuntary reflexes (Maynard et al., Annual Review of Neuroscience, 2020,4:316-36).

Dysphagia is a medical term to design any swallowing disorder, namely any dysfunction in one or more phases of the swallowing process. Dysphagia can be characterized by several symptoms such as cough before or after swallowing, choking, hoarse voice, regurgitations, persistent drooling of saliva, aspiration, food penetration, and improper chew. Patients with dysphagia often complain about sensation of food sticking or blocking in the throat or in the chest and/or chest or throat discomfort. Sometimes, dysphagia can be silent (i.e. cannot be identified by physicians without instrumentation) while causing serious health complications.

Indeed, malnutrition and dehydration, aspiration pneumonia, compromised general health, chronic lung disease, choking, and even death are possible consequences of dysphagia.

As of today, in view of its incidence in the population and its related morbidity, dysphagia is a major health concern.

In that matter, it was recently shown that dysphagia has a high prevalence in patients having autonomic nervous system dysfunction, e.g. in patients with certain neurodegenerative diseases or in patients suffering from some neurodevelopmental disorders such as Prader Willi syndrome.

Prader Willi Syndrome (PWS) is a complex genetic neurodevelopmental disorder that arises from loss of expression of paternally inherited imprinted genes on chromosome 15q11-q13 and drives hypothalamic dysfunction.

From birth to 9 months, infants with PWS display suckling disorders and anorexic behavior, combined with severe hypotonia. Conversely, after 2 years, young children with PWS subsequently demonstrate early excessive weight gain leading to severe obesity with hyperphagia and satiety deficit.

At all ages, the phenotype comprises also endocrine disorders (growth hormone deficiency, hypogonadism, hypothyroidism), learning disabilities, and behavioral and psychiatric disturbances. Patients with PWS also show several clinical manifestations such as oropharyngeal and bowel dysmotility, abnormal temperature regulation, altered sleep control, insensitivity to hypoxia and hypercarbia, altered perception of pain, decreased salivation, which evidence a dysfunction in the autonomic nervous system (ANS) (Haqq et al., 2012, clinical obesity 1, 175-183).

Although suckling deficit are well documented in infants, swallowing disorders in children and adults with PWS have not been assessed until recently in spite of the high incidence of pulmonary infections and sudden death in this population.

A recent study demonstrates that silent (subclinical) dysphagia is highly prevalent in children and adults with PWS. This study performed by videofluoroscopy of swallowing (VFS) showed that children and adults with PWS have disordered pharyngeal and esophageal swallowing with disturbances in timing, clearance, and coordination of swallowing with the respiratory cycle. All patients enrolled in the study had esophageal stasis and most of them had pharyngeal residues without being aware of it (Gross, et al. American Journal of Genetics, 2016, part A, 1-11).

Gross et al. (supra) is a study concerning the occurrence and the manifestation of dysphagia in subjects suffering from PWS.

Tauber et al. (2017, Pediatrics, 139, 2 e2 0162976) is a scientific publication concerning a phase II clinical trial assessing the effects of intranasal administration of OT on feeding and social skills in infants with PWS under 6 months old.

Patent application US 2020/0093884 relates to stable aqueous compositions comprising high concentrations of carbetocin, in particular for the treatment of hyperphagia behavior and obsessive and compulsive disorders in subjects with PWS.

Patent application US 2020/0093885 relates to a method for making improved carbetocin drug products.

As of today, there are very few options to manage dysphagia, in particular in patients with ANS dysfunction. The existing therapeutic approaches aim at safely supporting adequate nutrition and hydration while minimizing the risk of chocking and pulmonary infections. These approaches are mainly based on diet modifications and compensatory techniques such as maneuvers, postural/position techniques, biofeedback and oral-motor exercises. Anti-reflux and prokinetic drugs can be also administered in certain cases. However, these approaches are not curative and can be impossible to implement in patients with cognitive impairments or intellectual disabilities. Unfortunately, if the patient's swallowing safety and efficiency cannot reach a level of adequate function, or if swallow function does not support nutrition and hydration adequately, the sole remaining option is tube feeding e.g., nasogastric tube or gastrostomy.

As of today, there is a need for new therapeutic methods for managing dysphagia, in particular in subjects with ANS dysfunction.

### Summary of the invention

The invention is as defined in the appended set of claims.

The invention relates to the use of an oxytocin receptor agonist in the treatment or prevention of dysphagia in a subject of at least one year old and suffering from a neurodevelopmental disorder..

In some embodiments, the dysphagia is characterized by esophageal dysmotility. The dysphagia may be related to solids and/or liquids, preferably both.

In an embodiment, the subject suffers from a hypothalamic dysfunction.

In some embodiments, the neurodevelopmental disorder is a genetic neurodevelopmental disorder, preferably selected from the group consisting of Prader-Willi syndrome and Prader-Willi like syndromes, fragile-X syndrome, DiGeorge/22q11.2 Deletion Syndrome, Down syndrome, Rett syndrome, Noonan syndrome, CHARGE syndrome, Kabuki syndrome, Troyer syndrome, Christianson syndrome, Smith-Magenis syndrome, Alstrom syndrome, syndromic obesity, familial dysautonomia and Williams syndrome. In other embodiments, the neurodevelopmental disorder is selected from the group consisting of autistic spectrum disorder, cerebral palsy, intellectual disability, fetal alcohol spectrum disorder (FSAD) .

In a preferred embodiment, the subject is under food diversification transition or is already under diversified diet. The subject may be a toddler, a child or a teenager. The subject may be an adult as well.

In some embodiments, the dysphagia is characterized by at least one of the following clinical signs:
- esophageal stasis
- esophageal residues,
- abnormal closer of esophageal sphincter, in particular upper esophageal sphincter
- esophageal distention preferably upper esophageal distention or megaesophagus
- penetration, inhalation, including silent inhalation,
- decreased peristalsis,
- prolonged esophageal transit and
- esophageal reflux
and wherein said clinical signs are optionally evaluable by videofluoroscopy

In some embodiments, the subject has esophageal dysmotility characterized by one or several of the following features: a slow esophageal transit, a poor esophageal clearance, esophageal reflux, a decreased peristalsis and combinations thereof.

In some embodiments, the oxytocin receptor agonist is used to improve or normalize esophageal motility in the subject.

In some additional embodiments, the subject has experimented, experiments or is at risk of experimenting one or several of the following disorders: esophageal food bolus obstruction, pulmonary aspiration, recurrent pulmonary infection, aspiration pneumonia, choking, regurgitations, nasal regurgitation and meryscism.

Oxytocin receptor agonist of interest encompasses oxytocin, carbetocin, [Thr⁴]OT, HO[Thr⁴]OT, [Thr⁴, Gly⁷]OT, HO[Thr⁴, Gly⁷]OT, Lipo-oxytocin-1 (LOT-1), demoxytocin, merotocin, demoxytocin, lipo-oxytocin-1 (LOT-1), TC OT 39, and WAY-267464. LIT-001. Preferred oxytocin receptors agonists are oxytocin, carbetocin and combinations thereof. In some embodiments, the invention relates to the use of oxytocin or a pharmaceutically acceptable salt thereof in the treatment of dysphagia in a subject of at least one year old suffering from a neurodevelopmental disorder. The neurodevelopmental disorder is preferably Prader-Willi syndrome or a Prader-Willi like disorder. The subject may suffer from silent dysphagia. Oxytocin can be administered by intranasal route, preferably at a daily dosage from 2 IU to 48 IU.

### Figures

Figure 1 shows the different steps of the clinical study (see Example section).
Figure 2 shows the videofluoroscopy scoring chart for swallowing and oesophageal transit used during the clinical trial.

### Detailed description of the invention

The references to methods of treatment in this description are to be interpreted as references to the compounds, pharmaceutical compositions and medicaments of the present invention for use in a method for treating a subject by therapy.

As explained in the above background section, Prader Willi Syndrome (PWS) is a complex genetic neurodevelopmental disorder characterized by several distinct nutritional stages including the neonate phase in which the infant with PWS shows suckling deficits, anorexia and failure-to-thrive and subsequent phases in which the child or the adult shows excessive weight gain leading to obesity with hyperphagia and deficit of satiety. Subjects with PWS also show several clinical manifestations such as oropharyngeal, esophageal and bowel dysmotility, abnormal temperature regulation, altered sleep control, insensitivity to hypoxia and hypercapnia, altered perception of pain, decreased salivation, which strongly suggest a dysfunction in the autonomic nervous system (ANS).

High incidence of pulmonary infections and sudden death has been reported in PWS population as compared to healthy subjects and patients with similar intellectual disabilities. Recent studies have demonstrated that adults and children with PWS suffer from silent dysphagia characterized by esophageal statis, pharyngeal residues, high risk of silent aspiration and disturbances in coordination of swallowing with the respiratory cycle.

The Applicants performed systematic videofluoroscopy (VFS) in children with PWS and confirmed the high rate of dysphagia and risk factors for aspiration in this population. Thus, the Applicants sought therapeutic options to manage dysphagia in patients of PWS, and more generally in subjects having an ANS dysfunction.

Oxytocin (OT) is a naturally occurring hypothalamic hormone. It has peripheral hormonal actions and acts as a neurotransmitter and neuromodulator in the brain via an OT receptor. OT plays an important role in modulating social interactions and mother-infant bonding. Synthetic OT is marketed under the tradename Syntocinon^{®} or Pitocin^{®} for inducing labor, postpartum care and for enhancing lactation. OT have been assessed or is under evaluation in several clinical trials, e.g. for the treatment of social deficits in subjects with autism or for the treatment of hyperphagia and compulsive disorders in adults with PWS. The Applicants have recently completed a phase II clinical trial showing that the intranasal administration of OT is well-tolerated and significantly improves feeding and social skills in infants with PWS under 6 months old (Tauber et al., 2017, Pediatrics, 139, 2 e2 0162976).

However, to the knowledge of the Applicants, a possible effect of OT on dysphagia in children and adults, in particular with PWS, has never been reported or suggested in the prior art.

As shown in the example section, the Applicants demonstrated that oxytocin is effective in the management of dysphagia in subjects with ANS dysfunction.

In particular, the Applicant showed that the daily administration of oxytocin over three weeks enabled to improve esophageal transit with a complete normalization of peristalsis and upper esophageal sphincter closure in a 6 years old patient with Prader Willi syndrome having strong clinical symptoms of dysphagia.

Interestingly, the Applicants also showed the efficacy of the daily administration of oxytocin in a 14 years old patient presented hypothalamic disorder associated with a neurodevelopment disorder and suffering from dysphagia. The treatment with oxytocin enabled to normalize oropharyngeal initiation and synchronization as well as esophageal transit.

Furthermore, the Applicants are carrying out a clinical trial aiming at assessing the effect of chronical administration of oxytocin (OT) in PWS children and adolescents from 2 to 14 years old on dysphagia.

Based on these data, the Applicants are of the opinion that oxytocin can improve the swallowing pattern in the PWS subject by improving oro-pharyngo-esophageal motility as well as in other patients with autonomic nervous system dysfunction.

Thus, the Invention relates to the use of an agonist of oxytocin receptor in the treatment or the prevention of dysphagia, in particular in a subject suffering from an autonomic nervous system dysfunction in the context of a neurodevelopmental disorder such as PWS.

The Invention also relates to a pharmaceutical composition comprising an agonist of oxytocin receptor for use in the treatment or the prevention of dysphagia, in particular in a subject suffering from an autonomic nervous system dysfunction in the context of a neurodevelopmental disorder, e.g. in a subject with PWS.

In a particular aspect, the Invention relates to the use of an oxytocin receptor agonist or a pharmaceutical composition thereof for treating oro and/or pharyngo and/or esophageal dysmotility and/or swallowing-respiration desynchronization in the subject with dysphagia. In an additional aspect, the Invention relates to the use of an oxytocin receptor agonist or a pharmaceutical composition thereof for treating or decreasing the occurrence of a dysphagia symptom in the subject, said dysphagia symptom being selected from the group consisting of pharyngeal residues, nasal and/or esophageal regurgitations, aspiration, penetration, propulsive disorders and combinations thereof.

In another aspect, the Invention relates to the use of an oxytocin receptor agonist or a pharmaceutical composition thereof for preventing respiratory infections, including aspiration pneumonia, and/or chocking in the subject with dysphagia and with a neurodevelopmental disorder such as PWS.

As further detailed below, the subject is of at least one year old.

The subject is preferably under diversified diet or is under diet diversification.

In the context of the Invention, the subjects with dysphagia are patients with neurodevelopmental disorders, in particular genetic neurodevelopmental disorders such as PWS and PW-like disorder.

Detailed description of the invention is provided just below:

### • Definitions

As used herein, *"The treatment of dysphagia"* or *"treating dysphagia"* includes curing, delaying, alleviating or slowing the progression of dysphagia or that of one or more of associated symptoms and/or disorders as well as the prevention, the attenuation, the slowing, the reverse or the elimination one or more of the symptoms or disorders associated with dysphagia. For instance, in the context of the invention, the oxytocin receptor agonist can be used for attenuating or alleviating one or several manifestations of dysphagia in the subject such as aspiration, including silent aspiration, oropharyngeal dysmotility and esophageal dysmotility, propulsive troubles, desynchronization with the respiratory cycle, esophageal reflux and/or nasopharyngeal reflux, prolonged esogastric transit, and/or esophageal or pharyngeal residue.

The *"prevention of dysphagia"* includes preventing, or delaying the onset of dysphagia or one or more symptoms or complications associated with dysphagia. In some embodiments, this term also refers to minimizing the risk (or the probability) for a patient to develop said symptom or complication, as compared to a patient who has not been administered the compound of the invention. For instance, in the context of the invention, the oxytocin receptor agonist may be used to prevent or minimize the risk of experimenting respiratory infections, such as aspiration pneumonia, or chocking in a subject with dysphagia who may further suffer from a neurodevelopmental disorder. As a further example, the oxytocin receptor agonist can be used to delay the onset or slow-down the progression of dysphagia in a subject suffering from a neurodegenerative disorder.

As used herein, by *"a therapeutically effective amount"* is meant an amount of an oxytocin receptor agonist which prevents, removes, slows down, reduces, treats or delays one or several symptoms or complications of dysphagia in the subject.

The term *"pharmaceutically acceptable"* refers to compositions, compounds, salts and the like that are, within the scope of sound medical judgment, suitable for contact with the tissues of the subject, or which can be administered to the subject, without excessive toxicity or other complications commensurate with a reasonable benefit/risk ratio.

### • Dysphagia

As used herein, the term *"dysphagia"* refers to any swallowing disorder, namely any dysfunction in one or more phases of the swallowing process, namely in the oral, pharyngeal, and/or esophageal phases.

As explained in the background section, swallowing in adults and children is a complex mechanism based on a coordinated operation of the mouth, pharynx and esophagus. Swallowing mechanism includes three phases, namely the oral, pharyngeal, and esophageal phases.

In the context of the invention, the subject can suffer from oropharyngeal dysphagia, esophageal dysphagia or oropharyngo-esophageal dysphagia, depending on the phase(s) in which the swallowing disorder(s) occur(s).

In preferred embodiments, the subject suffers from oropharyngo-esophageal dysphagia Oropharyngeal dysphagia generally refers to disorders occurring in the oropharynx and the very upper part of the esophagus (around the upper esophageal sphincter) while esophageal dysphagia refers to disorders occurring in the esophagus (including the lower esophageal sphincter). Oropharyngo-esophageal dysphagia refers to disorders occurring both in the oropharynx and the esophagus.

Indeed, disorders can occur in each swallowing phase. For instance, L. van den Engel-Hoek et al. (Journal of Neuromuscular Diseases 2 (2015) 357-369) describes the following possible problems in each phase (list non-exhaustive):
- in the oral (preparatory) phase : Inadequate lip closure and disturbed tongue movements result loss of food from the mouth and mastication problems, problems with bolus formation and prolonged oral transport due to reduced tongue strength, which may lead to a delay in oropharyngeal initiation,
- in the pharyngeal phase : Pooling in the pharynx due to reduced tongue elevation, poor intraoral bolus control, and poor posterior tongue propulsion causing a delay in the initiation of pharyngeal swallow, nasal regurgitation due to incoordination of pharyngeal contractions or insufficient closure of the nasopharyngeal area, penetration of food underneath the epiglottis or (silent) aspiration due to timing and coordination deficits, residue after swallow in the valleculae and piriform sinuses caused by reduced activity of the submental muscle group, reduced tongue base retraction, or reduced pharyngeal contractions (pharynx dysmotility).
- in the esophageal phase : Residue above the upper esophageal sphincter due to limited opening of the sphincter, residue or pooling in the proximal esophagus due to motility problems.

In a particular embodiment, the subject suffers from a dysphagia related to oropharyngo-esophageal dysmotility (OPOD).

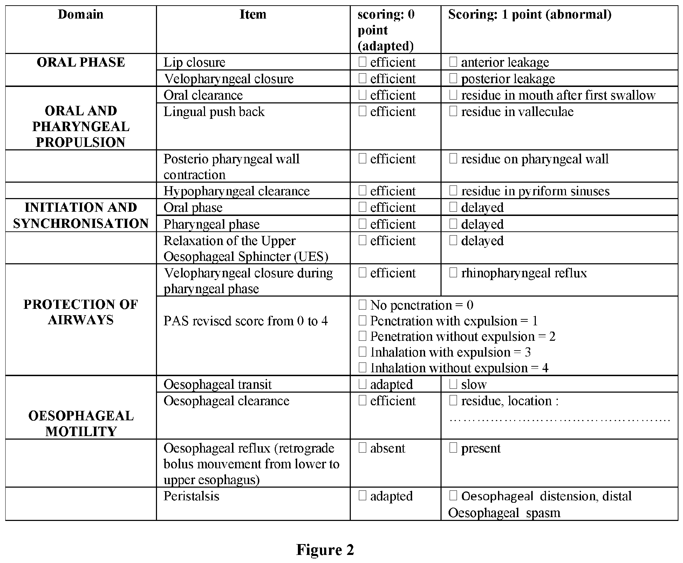

In a particular embodiment, the subject with dysphagia may exhibit one or several dysfunctions in the oral and pharyngeal propulsion, in the initiation and synchronization and/or in the esophageal motility, as listed in the chart shown in Figure 2.

In the context of the invention, the dysphagia can be related to solids and/or liquids, preferably both, i.e. the subject show swallowing disorders with both liquids and solids.

The subject with dysphagia can complain about sensation of food sticking or blocking in the throat or in the chest and/or chest or throat discomfort. The subject may suffer from visible or directly observable manifestations such as cough before or after swallowing, choking, hoarse voice, regurgitations, persistent drooling of saliva, improper chew, shortness of breath, sore throat, and heartburn.

However, in some subjects, the dysphagia can be silent, i.e. the dysphagia does not have visible manifestations in the subject and the subject does not feel any sensation relating to swallowing disorder. For example, the subject does not experiment any sensation of food sticking or blocking in the throat or the esophagus and/or fail to recognize the presence of aspiration and penetration, even if severe aspiration/penetration occurs. This may be due to associated cognitive problems or impaired sensory perception e.g. impaired pain perception as in the case of subjects with Prader-Willi syndrome

As used herein, "aspiration" describes a swallowing disorder wherein food or fluids or saliva that should go into the stomach go into the airway or the lungs instead. When this happens the subject still breathes but generally cough in order to clear the food or fluid out of their lungs. However, sometimes, the subject is not conscious that aspiration occurs and does not cough at all. Such a case is known as a "silent aspiration."

As used herein, "choking" describes a swallowing disorder wherein the food is lodged in the airway and blocks airflow. Sometimes, Heimlich maneuver is needed to expel the lodged food from the airway.

In some embodiments of the invention, the subject with dysphagia has chest or throat discomfort, including sensation of food sticking, and/or have one or several signs of dysphagia selected from the group consisting of cough before or after swallowing, choking, hoarse voice, regurgitations including merycism, persistent drooling of saliva, improper chewing, recognized aspiration or penetration, and heartburn.

In other embodiments, the subject suffers from silent dysphagia.

In the context of the invention, the term "dysphagia" generally refers to *"functional dysphagia",* which means that from an etiological point of view, the dysphagia is not explained by a structural, mucosal, or tissue lesion or abnormality such as head or neck tumors, Zenker's diverticulum, luminal stenosis, surgical resection of the oropharynx/larynx, radiation injury, anterior cervical spine surgery or from any extrinsic compression (e.g. goiter, cervical osteophyte or vascular compression of the esophagus as in the case of *Dysphagia lusoria*)*.* In particular, the term "dysphagia" in the context of the invention does not encompass *Dysphagia lusoria* or *Dysphagia* aorta.

In the context of the invention, the term *"dysphagia"* does not encompass dysphagia caused by diseases impairing the connective tissue such as autoimmune connective tissue diseases e.g. scleroderma as well.

The very exact mechanism of functional dysphagia is unknown but it may be related to inappropriate perception and muscle motility impairment in the swallowing apparatus or dysfunction of central integrating centers.

Furthermore, in some embodiments, the subject does not have any severe congenital malformation in the swallowing apparatus which encompasses the oral cavity including the mouth, the lips, and the tongue, the pharynx, the airway, and the esophagus and its sphincters, both upper and lower.

However, in certain embodiments, the subject may display some minor congenital anomalies in the swallowing apparatus as compared to a healthy subject, such as an ogival or high-arched palate, or a minor facial asymmetry. Besides long-lasting dysphagia in the subject can result in alterations in one or several parts of the swallowing apparatus. For instance, overtime, PWS subjects with long-lasting dysphagia can develop a mega esophagus.

Autonomic nervous system (ANS) dysfunction
can have a contribution in dysphagia, e.g. by impairing the oropharyngeal and esophageal motility, the synchronization between swallowing and respiratory cycle, the sensory perception and the integration and control of information in central integrating centers. The dysphagia in a subject may result, at least partially, from a disorder caused by an ANS dysfunction preferably selected from the group consisting of oropharyngeal and/or esophageal dysmotility, desynchronization between respiratory and swallowing cycles, impaired perception in the swallowing apparatus and central integrating centers and combinations thereof.

As used herein, *"dysmotility"* refers to any abnormality in the contractions/relaxations and in the coordination of muscle activities, of the oral cavity, and/or the pharynx, and/or the esophagus. The term "dysmotility" can encompass an abnormal pharyngeal and/or esophageal peristalsis, in particular a decreased esophageal peristalsis.

As mentioned above, the subject with dysphagia may suffer from esophageal dysmotility which can be characterized by one or several of the following features: a slow esophageal transit, a poor esophageal clearance (e.g. evidenced by the presence of esophageal residues), esophageal reflux (e.g. retrograde bolus movement from lower to upper esophagus), an abnormal peristalsis and combinations thereof.

Dysphagia can be diagnosed and explored by several techniques such as esophageal manometry, X-Ray examination with a contrast material (barium X-ray) by videofluoroscopy (VFS) or radiography, fiber-optic endoscopic evaluation of swallowing (FEES), pH monitoring, endoscopy such as pharyngoscopy or esophagoscopy, oesogastric transit, and imaging scans.

Of note, videofluoroscopy (VFS) is the gold standard method to study the oral, pharyngeal and esophageal mechanisms of dysphagia in a dynamic manner. VFS has been used by the Inventors to characterize dysphagia and assess the efficacy of oxytocin in patients suffering from Prader-Willi syndrome, as exemplified in the Example.

Indeed, VFS enables to characterize the major signs of oropharyngeal dysfunction, in particular delay in pharyngeal swallow, aspiration, nasopharyngeal regurgitation, esophageal dysmotility and pharyngeal residue.

To quantify the dysphagia in a subject by VFS, one may use e.g. the videofluoroscopy scoring chart for swallowing and oesophageal transit showed in Figure 2.

In some other or additional embodiments, the dysphagia is characterized by one or several clinical signs as follows:
- oropharyngeal dysmotility and/or esophageal dysmotility, in particular pharyngeal and/or esophageal stasis
- abnormal closer of esophageal sphincter, in particular upper esophageal sphincter,
- esophageal distention, preferably upper esophageal distention, or megaesophagus
- propulsive troubles,
- desynchronization with the respiratory cycle,
- esophageal reflux and/or nasopharyngeal reflux,
- aspiration including silent aspiration
- penetration, e.g laryngeal penetration of food
- prolonged esogastric, e.g. esophageal transit, and/or
- esophageal or pharyngeal residue.

Such clinical signs are preferably evidenced by VFS.

In a preferred embodiment, the subject with dysphagia display at least one (e.g. 1, 2, 3, 4, 5 and even all) of the following clinical manifestations:
- oropharyngeal dysmotility and esophageal dysmotility, in particular pharyngeal and/or esophageal stasis
- desynchronization with the respiratory cycle,
- nasopharyngeal and/or esophageal reflux,
- aspiration including silent aspiration
- penetration, e.g laryngeal penetration of food and
- esophageal or pharyngeal residue.

Said clinical manifestations can be evidenced by VFS.

In a particular embodiment of the invention, the dysphagia can be characterized by a dysmotility afflicted the oral cavity, and/or the pharynx and/or the esophagus. For instance, the dysphagia may be characterized by both oropharyngeal dysmotility and esophageal dysmotility, such dysmotility being preferably evidenced by VFS and/or manometry study. In another embodiment, the dysphagia can be characterized by a slow pharyngeal transit e.g. with pharyngeal residues, a slow esophageal transit e.g. with esophageal residue, esophageal reflux, an abnormal upper esophageal sphincter closure, a delayed oropharyngeal initiation and synchronization, and combinations thereof. It goes without saying that the administration of oxytocin receptor agonist in said patients enable to attenuate and even treat at least one of the above clinical manifestations.

For instance, the oxytocin receptor agonist may attenuate, prevent or treat a dysphagia symptom selected from the group consisting of aspiration, including silent aspiration, oropharyngeal dysmotility, esophageal dysmotility, esophageal stasis, abnormal upper esophageal sphincter closure, pharyngeal residues, regurgitations, desynchronization with the respiratory cycle, propulsive troubles and combinations thereof.

For instance, the oxytocin receptor agonist can be used to improve or normalize the esophageal motility, the oral and pharyngeal propulsion and/or the oropharyngeal initiation and synchronization in the subject with dysphagia. As illustrated in the case reports, the oxytocin receptor agonist can be used to improve and even normalize esophageal motility e.g. esophageal transit and peristalsis, upper esophageal sphincter closure and/or pharyngeal transit.

Even when silent, dysphagia can result in serious health problems and complications, in particular due to aspiration, and more particularly due to silent aspiration.

In some embodiments, the subject with dysphagia experiments or is at risk of experimenting dysphagia complications selected from the group consisting of esophageal food bolus obstruction, chronic respiratory infections including recurrent pulmonary infections, exacerbation of chronic obstructive pulmonary disease (COPD) and aspiration pneumonia, choking, sudden death and combinations thereof.

Dysphagia, in particular when associated with silent aspiration, can also increase the risk of sudden death by chocking.

In another aspect, dysphagia can lead to malnutrition, dehydration weight loss, as well as failure-to-thrive in children.

The administration of the oxytocin receptor agonist in the subject can treat or prevent one or several of the above listed complications, in particular respiratory infections, included aspiration pneumonia, and chocking.

### • Autonomic nervous system dysfunction and disorders with dysphagia pattern of interest

It is described that a subject with dysphagia may further display a dysfunction or a dysregulation in the Autonomic Nervous System (ANS).

The autonomic nervous system (ANS), formerly the vegetative nervous system, is a division of the peripheral nervous system that supplies smooth muscle and glands, and thus influences the function of internal organs. The autonomic nervous system is a control system that acts largely unconsciously and regulates bodily functions, such as the heart rate, digestion, respiratory rate, pupillary response, and urination as well as reflexes such as coughing, sneezing, swallowing and vomiting. The ANS includes peripheral and central neurons. It includes 1) afferent pathways which transmit sensory information, 2) integrating centers at the level for example of the brain stem (including the solitary tract), hypothalamus and limbic system and 3) efferent control pathways to muscles or glands.

The ANS can be separated into the sympathetic and parasympathetic nervous system. Some authors include the enteric nervous system in the ANS. Physiologically, both of the sympathetic and parasympathetic systems work at dynamic balance at rest as well as in response to stress.

By ANS dysfunction it is meant an alteration in the functioning of the autonomic nervous system which can cause one or several dysregulations in body functions. Dysfunction of ANS can lead to a variety of serious problems exhibited in multiple organ systems, such as tachycardia, sleep disorders, dysphagia, low blood pressure, orthostatic hypotension and others.

Dysautonomia refers to disorders which can affect all or part of the ANS. Dysautonomia can cause a variety of troubles such blood pressure problems, breathing troubles, or dysphagia and may be inherited or caused by injury or by conditions such as diabetes, neurodegenerative disease such as Parkinson disease, autoimmune disease, alcoholism and Riley-Day syndrome, also called familial dysautonomia.

It is described that dysphagia can be a symptom or a complication of a dysautonomia, such as a neurodegenerative disorder, in the subject.

Neurodegenerative disorders encompass, without being limited to, amyotrophic lateral sclerosis, muscular dystrophy, multiple sclerosis, Parkinson's disease, Alzheimer's disease, Lewy Body dementia, frontotemporal dementia, vascular dementia, Huntington's disease, progressive supranuclear palsy, multi-system atrophy, corticobasal degeneration and Creutzfeldt-Jakob disease.

Autonomic nervous system dysfunction can be also found in conditions which are not classified, or which have not been yet classified as, dysautonomia.

Autonomic nervous system dysregulation has been evidenced in various neurodevelopmental disorders such as Autism Spectrum Disorder (ASD), Rett syndrome and Prader Willi Syndrome (PWS).

On the other hand, dysphagia is a frequent complication in neurodevelopmental disorders which can be observed during the perinatal period but also later. Such a fact underlies that the genetic network relating to the sucking, feeding and swallowing (SFS) mechanism in infants and the transition to the feeding and swallowing in children/adults is vulnerable to genetic mutation and environmental disruption. The Applicants are of the opinion that ANS dysregulation or dysfunction can play a role in dysphagia observed in neurodevelopmental disorders e.g. by impairing the motility of the oropharynx and the esophagus, the synchronization between swallowing and respiratory cycle and the sensory perception in the swallowing apparatus or central integrating centers.

Accordingly, in the context of the invention, the subject with dysphagia suffers from a neurodevelopmental disorder. Thus, the dysphagia can be a symptom or a complication of a neurodevelopmental disorder in the subject.

As used herein, neurodevelopmental disorder refers to a group of disorders with severely affected behavioral features caused by alterations in early brain development. Most neurodevelopmental disorders are characterized by an alteration of the nervous system development and maturation affecting neuronal cell functions and the building of neuronal networks both required to i) sensory information perception and integration ii) the construction and coordination of adapted responses iii) the control of effectors (i.e. motor and endocrine responses) and iv) the building and maintaining of functional neuronal networks involved in learning and memory processes. Neurodevelopmental disorders lead to brain dysfunctions such as impairments in sensory or motor systems, speech, language or more generally orality alterations, a number of cognitive impairments (e.g. in learning, memory, organizational and/or planification skills), or combination.

Without to be bound by any theory, a hypothalamic dysfunction can contribute to dysphagia in the subject.

In some more particular embodiments, the subject suffers from a neurodevelopmental disorder involving or associated with hypothalamus dysfunction.

Such neurodevelopmental disorders are described further below and encompass e.g. Prader-Willi syndrome and Prader-Willi like syndromes, and Fetal alcohol spectrum disorder (FSAD).

The term "Neurodevelopmental" disorder encompasses both genetic and non-genetic neurodevelopmental disorders.

In some embodiments, the subject suffers from a non-genetic neurodevelopmental disorder, namely a neurodevelopmental disorder from which no genetic cause has been identified. In such a case, the neurodevelopmental disorder can be due to environmental disruption, e.g. exposure to alcohols, drugs, and other toxics such as pesticides in utero. In some other embodiments, the neurodevelopmental disorder is idiopathic or congenital without any clear origin and is suspected to be caused by a combination of genetic, and/or environmental and/or epigenetic factors.

Non-genetic neurodevelopmental disorders with dysphagia pattern encompass, without being limited to, intellectual disability, autism spectrum disorder (ASD), Fetal alcohol spectrum disorder (FSAD) including fetal alcohol syndrome, neonatal abstinence syndrome, cerebral palsy, and congenital heart disease.

In another embodiment, the subject suffers from a genetic neurodevelopmental disorder, namely a neurodevelopmental disorder caused by aneuploidy, disomy, chromosomal deletion, gene mutation and/or epigenetic alteration affecting for example genomic imprinting.

Genetic neurodevelopmental disorders with dysphagia pattern encompass without being limited to Prader-Willi syndrome and Prader-Willi like syndromes, fragile-X syndrome, DiGeorge/22q11.2 Deletion Syndrome, Down syndrome, Rett syndrome, Noonan syndrome, CHARGE syndrome, Kabuki syndrome, Troyer syndrome, Christianson syndrome, Smith-Magenis syndrome, Alstrom syndrome, syndromic obesity, familial dysautonomia and Williams syndrome. Dysphagia can be also observed in patients suffering from genetic neurodevelopmental disorders such as Pierre Robin syndrome, Septo-optic dysplasia (SOD), and ROHHAD (rapid-onset obesity, hypothalamic dysfunction, hypoventilation, and autonomic dysregulation) syndrome.

In a preferred embodiment, the subject with dysphagia suffers from Prader Willi Syndrome (PWS) or PW-like disorder. More details about this sub-group of patients are provided further below.

### • The subject

The term "subject" or "patient" " refers to human subjects.

The subject suffering from dysphagia may be of any gender.

For instance, the subject may be a toodler, a child, an adolescent an adult including an elderly subj ect.

Of note, suckling in infants is very different from the feeding and swallowing in children and adults. This is due to the immaturity of the oropharyngeal area, more precisely to the high position of the larynx and the hyoid which allows for the immature epiglottis to latch against the back of the soft palate, creating separate channels for simultaneous nasal breathing and swallowing liquid. Thus, the suckling in infants is a continuous behavior, without any pause for breathing as in adult. Initially, the suckling is controlled by brainstem reflexes which disappear with age. Over the first year, the neck grows, the hyoid and larynx lower, the epiglottis flattens, and the soft palate matures, facilitating the transition to eating solid food between four and six months. The complete swallowing mechanism develops thereafter, by gradual changes in oropharyngeal muscle recruitment and pharynx growth (Maynard et al., Annual Review of Neuroscience, 2020,4:316-36).

In a preferred embodiment, the subject is under food diversification transition or is already under diversified diet, i.e. is able to eat both solid and liquid food. More preferably, the subject is not under liquid diet only.

The transition to eating solid food generally occurs between four and six months of age. Thus, the subject with dysphagia is preferably of at least 6 months old, e.g. of at least 9 months old or at least one year old.

The suckling-feeding-swallowing mechanism, an innate and reflex-driven behavior, then transitions to a voluntary behavior during infancy. The rooting reflex disappears in normal infants around 7 months of age. After 12 month old, suckling/swallowing reflex is totally lost and swallowing becomes a voluntary movement.

In other words, at the age of 12 month old, a child has underwent the morphologic and functional changes leading to a different swallowing mechanism.

Accordingly in the context of the invention, the subject is at least one year old.

In some embodiments, the subject is under 50 year old, for instance under 40, 30 or 20 year old.

In a particular embodiment, the subject is under 18 year old, preferably of at most 16 year old.

Typically, the subject with dysphagia is a child of 1 year old to 16 year old, preferably from 2 to 15 years old.

As mentioned above, the subject may suffers from a genetic neurodevelopmental disorder such as PWS or Prader-Willi like syndrome.

In some embodiments, the subject suffers from cognitive impairments and/or intellectual disabilities.

In some other or further embodiments, the subject suffers from poor oral skills.

In some alternate or additional embodiments, the subjects have suffered from feeding disorders in infancy, preferably during the neonate period. Said feeding disorders preferably refer to suckling deficits.

In some other embodiments, the subjects have not undergone a treatment with oxytocin during infancy, in particular within the first 6 months from birth.

### • Oxytocin receptor agonist of interest

The term "agonist" refers to any compound that interacts with a target to cause or promote an increase in the activation of said target in a selective manner. In the context of the invention, the target is the "oxytocin receptor", preferably "human oxytocin receptor".

Of note, a preferred oxytocin receptor agonist is oxytocin (OT) itself, a mammalian nonapeptide expressed in the hypothalamus and which can be easily synthesized by very well-known proceeding. The formula of oxytocin is as follows:

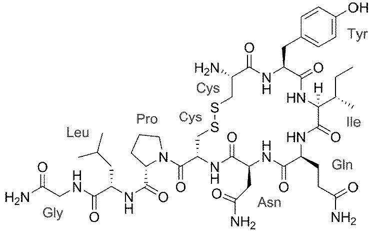

Alternatively, the oxytocin receptor agonist can be any compound able to activate oxytocin receptor, preferably in a selective manner. The compound may be of any type. It can be selected from chemical drugs preferably of at most 3000 g.mol⁻¹, antibodies and fragments thereof, and nucleic acid ligands such as aptamers.

As used herein, an "aptamer" (also called nucleic aptamer) refers to a synthetic single-stranded polynucleotide typically comprising from 20 to 150 nucleotides in length and able to bind with high affinity a target molecule. The aptamers are characterized by three-dimensional conformation(s) which may play a key role in their interactions with their target molecule.

As used herein, the term "antibody" refers to an immunoglobulin or a fragment or a derivative thereof, and encompasses any polypeptide comprising an antigen-binding domain, regardless whether it is produced in vitro or in vivo. The term includes, but is not limited to, polyclonal, monoclonal, monospecific, multispecific (e.g. bispecific), humanized, single-chain, chimeric, synthetic, recombinant, hybrid, mutated, and grafted antibodies. The term "antibody" also includes antibody fragments such as Fab, F(ab')2, Fv, scFv, Fd, dAb, and other antibody fragments (e.g. VHH from single-chain antibody) that retain antigen-binding function, i.e., the ability to bind their target specifically.

Various oxytocin receptor agonists are described in the prior art. Besides, the prior art also describes several in vivo or in vitro assay to assess whether a given molecule is an agonist of oxytocin receptor or not.

For instance, one can refer to Manning et al. (J. Neuroendocrinol, 2012, 24(4):609-628) which refer to binding and functional assays in cell lines expressing hOT receptor based on cyclic AMP accumulation. As another example, the agonist activity towards OT receptor may be measured by the method set out in WO2009/122285. In this method, agonist activity of compounds on the hOT receptor is determined in a transcriptional reporter gene assay by transiently transfecting a hOT receptor expression DNA into a Chinese Hamster Ovary (CHO) cell line in concert with a reporter DNA containing intracellular calcium responsive promoter elements regulating expression of firefly luciferase. Cells are exposed to serial dilutions of compounds diluted 10-fold per dose for 5 hours, followed by lysis of cells, determination of luciferase activity, and determination of compound efficacies and EC50 values through non-linear regression. Oxytocin (OT) is used as an internal control in each experiment, and compounds are tested in at least three independent experiments.

A more specific functional assay can be found in Busnelli et al. (The journal of Biological Chemistry, 2012, vol. 287, p. 3617-3629) in which functional selective ligands of the human OT receptor are screened using a BRET-based biosensor assay.For review concerning OT receptor agonists, one can refer, for instance to Manning et al., Journal of neuroendocrinology, 2012, 24,609-628.

In some embodiments, the oxytocin receptor agonist is selective to oxytocin receptor, more precisely to the human OT receptor which means that it does not significantly bind to and/or activate other receptors.

In some other embodiments, the oxytocin receptor agonist may be able to bind and even activate another receptor such as V1a, V1b and V2 receptors, but preferably with a lower potency than for the oxytocin receptor (e.g. with a EC50 which is at least 1.5, preferably at least 10, 20, 50 or 100-fold higher than the EC50 for the oxytocin receptor).

In a particular aspect, the invention also relates to the use of an agonist of V1a, V1b and/or V2 receptors which is also able to activate oxytocin receptor. Such compounds of interest encompass for instance vasopressin, argenine vasopressin, arginine vasotocin, vasotocin and analogues thereof.

In a preferred embodiment, the oxytocin receptor agonist is a synthetic chemical molecule. It can be a peptide or a non-peptide molecule preferably of at most 3000 or at mot 2000 g. mol-1.

Non-peptide agonists of oxytocin receptor are described e.g. in WO2014/111356 and include e.g. TC OT 39, LIT-001 and WAY-267464.

Preferably, the oxytocin receptor agonist is a peptide, in particular an analogue of oxytocin, i.e. a compound which has a chemical structure close to that of oxytocin. Analogues of oxytocin can differ from oxytocin in virtue of one or several amino acid modifications (e.g. by 1, 2, 3, 4, or 5 modifications selected from insertion, deletion or substitution, including with non-natural amino acids) or in virtue of one or several chemical modifications. Analogues of oxytocin are described for example in WO2016/044131, WO2011/035330 and WO2009/122285.

For illustration only, the agonist of the invention can be a compound of formula (I) as described in WO2016/044131 wherein 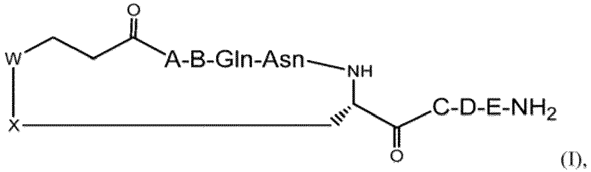 wherein
each of W and X, independently, is CH2 and S, provided that W and X are not both CH2; A is an amino acid selected from the group consisted of alanine substituted on the side chain with a 5- or 6-membered heteroaromatic ring; tyrosine; and phenylalanine substituted on the phenyl ring with halogen, C1-4 alkoxy, C1-4 alkylhydroxy, C1-4 alkyl or amino;
B is an amino acid selected from the group consisting of isoleucine; and glycine substituted on the a-carbon with C4-6 cycloalkyl;
C is an amino acid selected from the group consisting of proline, optionally substituted on the side chain with hydroxyl, C1-4 alkoxy, halogen or azide, and proline having its side chain optionally interrupted by a heteroatom and which optionally interrupted side chain is optionally substituted with C1-4 alkyl;
D is an amino acid selected from the group consisting of leucine; homoleucine; isoleucine; and glycine substituted on the α-carbon with C4-6 cycloalkyl; and
E is an amino acid selected from the group consisting of glycine and azaglycine. In some examples, W is CH2 and X is S. In some examples, A is phenylalanine substituted on the phenyl ring with C1-4 alkoxy.

In some embodiments, the oxytocin receptor agonist is selected from the group consisting of carbetocin, [Thr⁴]OT, HO[Thr⁴]OT, [Thr⁴, Gly⁷]OT, HO[Thr⁴, Gly⁷]OT, Lipo-oxytocin-1 (LOT-1), demoxytocin, merotocin (developmental code name FE-202767), pharmaceutically acceptable salts thereof and combinations thereof.

In some preferred embodiments, the oxytocin receptor agonist is selected from oxytocin, carbetocin, pharmaceutically acceptable salts thereof and combinations thereof.

### • Administration route, regimen, and formulation

The oxytocin receptor agonist may be administered by any conventional route including, but not limited to, oral, buccal, sublingual, intravenous, intra-muscular, intranasal, pulmonary (e.g. by inhalation), or subcutaneous route.

Indeed, depending on the agonist to be used, the skilled artisan is able to determine the best route to use, in particular in view of its bioavailability and its stability.

Preferred routes of administration are the intranasal and the oral one. A more preferred route of administration is the nasal/intranasal route.

As used herein, intranasal administration refers to the delivery of a drug in the nasal cavity. The effective dose of oxytocin receptor agonist to administer to the subject can be determined by the use of conventional techniques and by observing results obtained under analogous circumstances. The therapeutically effective dose may vary depending on the, genotype of the subject, the type of dysphagia and concomitant disorders, the clinical manifestations of dysphagia observed in the subject, the gravity of dysphagia, the evolution of dysphagia overtime, the route of administration, any co-therapy involved, the patient's age, weight, general medical condition, medical history, etc.

The daily amount of an oxytocin receptor agonist to be administrated to the subject is generally of at most 500 mg, for instance at most 400, 300, 200, 100, 50, 40, 30, 20, 10, 5, 1, 0.5 or 0.2 mg depending on its agonistic potency.

By convention, for the purpose of labelling of oxytocin-containing drug, oxytocin dosage is expressed in International Unit. 1 IU corresponds to about 1,667 µg of oxytocin (which is equivalent to say that 1 mg of oxytocin corresponds to 600 IU) (see OXYTOCIN: ADOPTED TEXT FOR THE INTERNATIONAL PHARMACOPOEIA, WHO, June 2010) In the context of the invention, the daily dose of oxytocin is generally lower than 100 IU (i.e. 166,7 µg), and preferably from 1 IU (1,667 µg) to 50 IU (83,35 µg).

For instance, the daily dose of oxytocin is typically from 2 IU to 50 IU, preferably from 4 IU to 32 IU, or from 4 IU to 24 IU.

As a further illustration, carbetocin can be administered at a daily dose of at most 30 mg, typically from 3 mg to 30 mg per day.

The oxytocin receptor agonist can be administered once daily, every two days, or several times daily, e.g. twice, three times or four times daily. The OT receptor agonist can be administered with or without food, preferably without food, for instance before meals. The administration of oxytocin receptor agonist can be performed during one or several weeks, or one or several months, and typically as long as it is necessary.

The oxytocin receptor agonist is typically formulated into a pharmaceutical composition to be administered to the subject. The oxytocin receptor agonist may be formulated in any appropriate pharmaceutical composition according to standard methods such as those described in Remington: The Science and Practice of Pharmacy (Lippincott Williams & Wilkins; Twenty first Edition, 2005). Pharmaceutically acceptable excipients that may be used are, in particular, described in the Handbook of Pharmaceuticals Excipients, American Pharmaceutical Association (Pharmaceutical Press; 6th revised edition, 2009). Typically, the therapeutic agent is mixed with one or several excipients so as to obtain the desired pharmaceutical form. Examples of appropriate excipients include, but are not limited to, solvents such as water or water/ethanol mixtures, fillers, carriers, diluents, binders, anti-caking agents, plasticizers, disintegrants, lubricants, flavors, buffering agents, stabilizers, colorants, dyes, anti-oxidants, anti-adherents, softeners, preservatives, surfactants, wax, emulsifiers, wetting agents, isotonic agent and glidants. Examples of diluents include, without being limited to, microcrystalline cellulose, starch, modified starch, dibasic calcium phosphate dihydrate, calcium sulfate trihydrate, calcium sulfate dihydrate, calcium carbonate, mono- or disaccharides such as lactose, dextrose, sucrose, mannitol, galactose and sorbitol, xylitol, glycerol and combinations thereof. Examples of binders include, without being limited to, starches, e.g., potato starch, wheat starch, corn starch; gums, such as gum tragacanth, acacia gum and gelatin; hydroxypropyl cellulose, hydroxyethyl cellulose, hydroxypropyl methyl cellulose; polyvinyl pyrrolidone, copovidone, polyethylene glycol and combinations thereof. Examples of lubricants include, without being limited to, fatty acids and derivatives thereof such as calcium stearate, glyceryl monostearate, glyceryle palmitostearate magnesium stearate, zinc stearate, or stearic acid, or polyalkyleneglycols such as PEG. The glidant may be selected among colloidal silica, dioxide silicon, talc and the like. Examples of disintegrants encompass, without being limited to, crospovidone, croscarmellose salts such as sodium croscarmellose, starches and derivatives thereof.

Examples of surfactants encompass, without being limited to, simethicone, triethanolamine, les polysorbate and derivatives thereof such as tween^{®} 20 or tween^{®} 40, poloxamers, fatty alcohol such as laurylic alcohol, cetylic alcohol, phospholipids and alkylsulfate such as sodium dodecylsulfate (SDS). Example of stabilizers, in particular useful for lyophilization encompass stabilizers typically encompass sugars such as mannitol, sucrose, dextrose and trehalose, amino-acids, hydroxypropyl-β-cyclodextrin and serum albumin. Examples of emulsifiers, encompass for example, ethyl alcohol, isopropyl alcohol, ethyl carbonate, ethyl acetate, benzyl alcohol, benzyl benzoate, propyleneglycol, 1,3-butyleneglycol, dimethylformamide, oils, polyethyleneglycol and fatty acid esters of sorbitan or mixtures of these substances. Preservatives encompass, without being limited to, parabens, chlorobutanol, benzalkonium chloride, benzoic acid, sorbic acid and salts thereof. Antioxidants encompass ascorbic acid, ascorbyl palmitate, tocopherol and combinations thereof. Examples of buffering agents encompass phosphoric acid, Tris(hydroxymethyl)aminomethane hydrochloride (TRIS.HCl), 4-Morpholinepropanesulfonic acid (MOPS), 4-(2-Hydroxyethyl)piperazine-1-ethanesulfonic acid (HEPES), PIPES, 2,2-Bis(hydroxymethyl)-2,2',2"-nitrilotriethanol (BIS-TRIS), TRIS-glycine, Bicine, Tricine, TAPS, TAPSO, MES, citrate, acetate, borate, citrate/phosphate, bicarbonate, glutaric acid, succinic acid, citric acid, acetic acid salts thereof and combinations thereof.

It goes without saying that the excipient(s) to be combined with the oxytocin receptor agonist of interest may be selected taking into account (i) the physico-chemical properties including the stability of said therapeutic agent, (ii) the pharmacokinetic profile and/or the release profile sought for said therapeutic agent, (iii) the dosage form and (iv) the route of administration.

The pharmaceutical composition may be of any type. For instance the pharmaceutical composition may be a solid oral dosage form e.g. as a tablet, a liquid dosage form e.g. for intranasal delivery, a suspension, for instance for intravenous route, a suppository, an aerosol e.g. for administration by pulmonary route... The pharmaceutical composition may be also a freeze-dried powder to be dissolved or suspended in an appropriate vehicle before administration, e.g. by intranasal route.

As mentioned, a preferred route of administration is the intranasal one.

Thus, in certain embodiments, the pharmaceutical compositions of the invention are those suitable for intranasal administration, such as nasal spray, or dry powder aerosol formulation. For example, a pharmaceutical composition of interest for intranasal delivery can be in the form of a liquid composition comprising the oxytocin receptor agonist of interest dissolved in an appropriate aqueous buffer. Appropriate buffer agents encompass phosphate salts, citric acid, acetic acid, acetate salts, aspartate salts and combinations thereof. The liquid composition can comprise one or several additional pharmaceutical excipients such as preservatives, antioxidants, surfactants, solvents, isotonic agents, pH adjustment agents (e.g. HCl or NaOH) and wetting agents. The pH of the composition can be adjusted between pH 4.5 to pH 6.0. Divalent ions such as Ca2+, Mg2+ or Zn2+ can be added for stabilization purposes or promoting the absorption in vivo.

A liquid composition of oxytocin for intranasal administration is marketed under the tradename Syntocinon^{®} in Europe. Syntocinon^{®} is an aqueous buffered formulation of oxytocin comprising a combination of buffering agents (e.g. phosphate, citric acid, and acetate) together with sodium chloride, sorbitol, glycerol and preservatives (chlorobutanol, methyl paraben and propyl paraben) in water.

The prior art also provides several examples of intranasal compositions which can be used for implementing the invention. For instance, one can refer to WO2012042371 which describes liquid compositions of oxytocin receptor agonists (e.g. carbetocin) comprising phosphate salt and citric acid as buffering agents and NaCl as isotonic agent in water at pH 5.5. The compositions may further comprise an antioxidant as methionine or EDTA. These compositions are described as suitable for nasal administration.

Other examples can be found in WO2016112205 which refers to liquid compositions for nasal administration comprising oxytocin and a magnesium salt. This international application specifically describes buffered aqueous composition of oxytocin comprising magnesium salt (e.g. MgCl), buffer agent such as acetate and citrate in water at pH 4.5. Additional oxytocin formulations are for instance described in Avanti et al (AAPS J. 2011 Jun;13(2):284-90 and Int J Pharm . 2013 Feb 28;444(1-2):139-45). Avanti et al. describe stable aqueous solutions of oxytocin obtained by using citrate or aspartate with divalent metal cations such as Ca2+, Mg2+ and Zn2+ (Int J Pharm 2013 Feb 28;444(1-2):139-45).

The intranasal composition of the invention can be administered as a single dose or in divided dosages, for example, into 1, 2, 3, 4, 5 or 6 sub-doses (e.g., puffs) delivered to one or both nostrils.

The intranasal composition of oxytocin receptor agonist can be delivered by any mean, e.g. by using any appropriate nasal pump apparatus or device. Such devices are well known by the skilled artisan and various models are available on the market. In some embodiments, the nasal apparatus or device comprises a reservoir bottle attached to a pump actuator. In some embodiments, the nasal apparatus or device comprises a reservoir bottle attached to an aerosolizer. The device for intranasal delivery is preferably designed for delivery of multiple doses of the oxytocin receptor agonist composition. For example, a nasal pump apparatus may comprise a reservoir bottle attached to a pump actuator where the reservoir bottle holds multiple doses of the liquid formulation and the pump actuator is metered to deliver a specified volume that is a fraction of the liquid formulation held in the reservoir bottle. In some embodiments, the pump actuator is metered to deliver about 5 µL to about 200 µL, preferably from 25 µL to 170 µL, e.g. about 40 µl to 150 µl of the liquid formulation per spray. The nasal pump apparatus may comprise a filter for preventing back flow in order to reduce or prevent contamination (e.g., bacterial) of the reservoir bottle.

For instance, the device for intranasal delivery may be equipped with a multi-dose pump comprising a microbial filter and an auto-blocking mechanism in the pump actuator, e.g. as described in U.S. Pat. No. 5,988,449.

### • Particular embodiment of the invention : Treatment of dysphagia in subjects suffering from Prader Willi Syndrome and related disorders

In a particular aspect, the invention relates to the use of an oxytocin agonist receptor in the treatment or the prevention of dysphagia in a subject suffering from Prader Willi Syndrome (PWS) or Prader Willi like syndrome.

Prader Willi Syndrome (PWS) is a complex genetic neurodevelopmental disorder that arises from lack of expression of paternally inherited imprinted genes on chromosome 15q11-q13 and drives hypothalamic dysfunction. The estimated incidence is 1/15,000 births to 1/27,000 births. The lack of gene expression can be due to chromosomal deletion, maternal disomy or an imprinting defect.

Clinical features of PWS include severe neonatal hypotonia with sucking deficit which explains the frequent failure to thrive (FTT) in the neonatal period and infancy (0 to 9 months), a global developmental delay with hypogenitalism/hypogonadism (2 to 6 years), development with hyperphagia leading to morbid obesity if not controlled (6-12 years), and then permanent obsession with food closed to addiction and lack of satiety, hypothalamic and pituitary dysfunction (short stature related to growth hormone deficiency, hypogonadism, hypothyroidism and rare central adrenal insufficiency), learning disabilities, cognitive deficit, behavioral problems including social skills deficit and psychiatric phenotypes 13 years through adulthood) (Gunay-Aygun et al. Pediatrics. 2001;108(5):E92). PWS is classically described as having two distinct nutritional stages. In Stage 1, the subject exhibits poor feeding and severe hypotonia, often with failure to thrive (FTT). Then, Stage 2 which begins around 2-4 years is characterized by a rapid weight gain, onset and escalation of hyperphagia leading to obesity, developmental delays and onset of behavior disorders. The switch from poor feeding/FTT to obesity/hyperphagia has not been fully elucidated and result from a complex evolution comprising several nutritional phases as shown in Miller et al. (Am J Med Genet A. 2011 May; 155A(5): 1040-1049). Miller et al. describe:
- Phase 1 wherein the infant is hypotonic and not obese, with sub-phase 1a characterized by difficulty feeding with or without FTT (median age at completion: 9 months). This phase is followed by sub-phase 1b when the infant grows steadily along a growth curve and weight is increasing at a normal rate (median age of onset: 9 months),
- Phase 2 which is associated with weight gain with in sub-phase 2a in which the weight increases without a significant change in appetite or caloric intake (median age of onset 2.0 years), while in sub-phase 2b, the weight gain is associated with a concomitant increased interest in food (median age of onset: 4.5 years),
- Phase 3 which is characterized by hyperphagia, typically accompanied by food-seeking and lack of satiety (median age of onset: 8 years), and
- Phase 4 s when an individual who was previously in phase 3 no longer has an insatiable appetite and is able to feel full.

As used herein, Prader-Willi like syndrome or Prader-Willi like disorder refers to any genetic neurodevelopmental disorder which recapitulates most of the clinical phenotypes of PWS. Prader-Willi like disorder encompass Schaaf Yang syndrome (pathogenic mutation in *MAGEL* 2 gene) and microdeletions in SNORD116 gene cluster. Deletion or mutation of the following genes SNURF-SNRPN, MKRN3 gene, *NDN* gene and several snoRNAs sequence, NPAP1gene, C15orf2 gene are also encompassed.

Other Prader-Willi like disorders are described in Rocha and Paiva (2014, Genetics and Molecular Research 13 (1): 2290-2298) and encompass chromosomal abnormalities such as a duplication of Xq, 1p36 monosomy, a 6q deletion, 10q26 deletion, 12q subtelomere deletions (Niyazov et al., 2007), chromosomal abnormality associated with Angelman's syndrome, and 2pter deletion, and deletion involving SIM1 gene.

In some embodiments, the oxytocin receptor agonist, preferably oxytocin or carbetocin, is used to attenuate, prevent or treat a dysphagia symptom in a subject with PWS or PWS-like disorder, said symptom being selected from the group consisting of aspiration, including silent aspiration, penetration, oropharyngeal dysmotility, esophageal dysmotility, pharyngeal residues, regurgitations, desynchronization with the respiratory cycle, propulsive troubles and combinations thereof.

In a preferred embodiment, the oxytocin receptor agonist, preferably oxytocin or carbetocin is used to treat oropharyngeal dysmotility, and/or esophageal dysmotility and/or respiratory-swallowing desynchronization and/or propulsive disorders in a subject with PWS of PW-like syndrome.

In another embodiment, the oxytocin receptor agonist, preferably oxytocin or carbetocin, is used to treat or prevent one or several of dysphagia complications selected from the group consisting of respiratory infections, included aspiration pneumonia, chocking and combinations thereof, in a subject with PWS or PWS-like disorder.

In a preferred embodiment, the subject to be treated is not a neonate or an infant under liquid feeding only. Preferably, the subject is under food diversification transition or is already under diversified diet, i.e. is able to eat both solid and liquid food.

The subject with PWS or PW-like syndrome is of at least 12 months old. Additionally, the subject with PWS of PWS-like syndrome may be at most 20 year old, preferably at least 15 year old.

In some embodiments, the subject has not been treated with oxytocin within 6 months from birth.

In other embodiments, the subject with PWS is in nutritional phase 1b, 2, 3 or 4 as described above, in particular in nutritional phase 1b, 2 or 3, more preferably in phase 2 or 3.

In another embodiment, the subject with PWS or PWS-like syndrome also suffers from hyperphagia.

As mentioned above, preferred OT receptor agonists are oxytocin, carbetocin and pharmaceutically acceptable thereof. More preferably, the OT receptor agonist is oxytocin of a pharmaceutically salt thereof.

The oxytocin receptor agonist is preferably administered by intranasal route, once a day or several times a day (e.g. twice or three times a day). Preferably the OT receptor agonist is administered once a day. The daily pharmaceutically dose of the OT receptor agonist is at most 100 IU, preferably from 2 IU to 50 IU.

Appropriate daily doses of oxytocin for subject of 2 to 15 year old are typically from 2 IU to 48 IU, preferably from 2 IU to 32 IU, from 4 IU to 20 IU such as 6 IU to 16 IU. Oxytocin may be administered once a day, e.g. in the morning, preferably before meals.

The daily dose of carbetocin is typically from 3 to 30 mg. Carbetocin may be administered three times a day, preferably before meals.

The OT receptor agonist, e.g. oxytocin or carbetocin, may be typically administered in the form of a buffered aqueous composition i.e. in the form of a nasal spray.

Further aspects and advantages of the present invention are disclosed in the following experimental section, which should be regarded as illustrative and not limiting the scope of the present application.

### EXAMPLE SECTION

### EXAMPLE 1: Case reports

PATIENT 1 was 6 years old with Prader-Willi syndrome due to maternal uniparental disomy diagnosed soon after at birth.

He displayed clinical symptoms of dysphagia with strong difficulty to swallow water, but not solid food, with acid reflux and dental problems possibly related to acid reflux episodes. The videofluoroscopic swallow study (VFSS) was abnormal with prolonged oesophageal transit and decreased peristalsis and abnormal closure of upper oesophageal sphincter.

He received 8IU of daily intranasal OXT during 3 weeks.

Treatment with oxytocin was well tolerated with improved VFSS comprising improved oesophageal transit with complete normalization of peristalsis and normal upper oesophageal sphincter closure.

PATIENT 2 was 14 years old. He suffered from neurodevelopmental disorder and presented hypothalamic disorder. He displayed clinical symptoms of dysphagia with frequent episodes of ruminating and bad breath.

The VFSS was abnormal including slow pharyngeal transit with pharyngeal residues and slow oesophageal transit with oesophageal reflux and stasis.

He received daily 16 IU of intranasal OXT during 3 weeks. The treatment was well tolerated and improved clinical symptoms of dysphagia with normalization of oropharyngeal initiation and synchronisation and normalization of oesophageal transit. After treatment, VFSS showed neither stasis nor reflux .

### EXAMPLE 2: EFFECT OF INTRANASAL OXYTOCIN ON DYSPHAGIA RELATED TO OROPHARYNGO-OESOPHAGEAL DYSMOTILITY IN CHILDREN AND ADOLESCENTS WITH PRADER-WILLI SYNDROME: A PHASE 3 STUDY (DYSMOT)

The aim of the present study is to investigate the effects of intranasal OT treatment on dysphagia related to OroPharyngo-Oesophageal Dysmotility (OPOD) in children aged 2 to 18 years with Prader Willi syndrome (PWS) using systematic videofluoroscopic swallow study (VFSS).

**Table 1: synopsis**

| | | |
|---|---|---|
| **Primary Study Objective** | To compare the effect of a 12-week intranasal administration of OT versus (vs) placebo on dysphagia related to OPOD assessed by videofluoroscopy of swallowing and oesophageal transit in children and adolescents with PWS aged 2 to 18 years. | |
| **Secondary Study Objectives** | To compare the effect of a 12-week intranasal administration of OT vs placebo on: | |
| | | *•* Swallowing symptoms; |
| | | *•* Feeding events |
| | To document the effect of 12 weeks OT treatment withdrawal on dysphagia related to OPOD. | |
| | To document the maintenance of efficacy over a 24 week-intranasal administration of OT. | |
| | To document the long-term efficacy up to 48-weeks of intranasal administration of OT. | |
| **Safety Objective** | To assess the overall safety of a 24-week and up to 48-week intranasal administration of OT. | |
| **Study Design** | | This prospective, multicentre, randomised, double-blind, Phase 3 clinical study is planned to include around 36 PWS patients aged 2-17 years and 5 months from 5 French centres (Toulouse, Rouen, Lille, Lyon and Marseille) with long term expertise on dysphagia. The study comprises 2 parts. |
| | | The first part is a placebo-controlled, double-blind period to assess the effects of a 12-week course of intranasal OT treatment vs placebo on dysphagia related to OPOD assessed by VFSS and oesogastric transit in male and female children and adolescents with PWS. Group 1 and 3 will receive OT while Group 2 will receive placebo. Patients will be randomised to 1 of the 3 treatment groups in a 1:1:1 ratio. Randomisation will be stratified by age group (2 to < 8 years, 8 to 15 years, >15). |
| | | In the second part, another double-blind period, Group 1 patients (treated with OT during the first part) will receive 12 weeks of placebo to assess the changes after stopping the treatment whilst Group 2 patients (treated with placebo during the first part) will receive OT. Group 3 (patients who received OT during the first part) will continue to receive OT. |
| | | In the third part, an open label period, all patients will receive 24 weeks of OT to assess long-term safety and maintenance of efficacy. |
| **Investigational Medicinal Product** | | **Formulation:** |
| | | OT: intranasal oxytocin solution (177,76 IU/mL) in amber glass bottle with a spray device with protective cap. |
| | | Placebo: a matching intranasal placebo solution in identical packaging with the same excipients as OT. |
| | | **Route of administration:** |
| | | Intranasal |
| | | **Dose regimen and duration:** |
| | | Study treatment will be administered intranasally daily for 48 weeks. The daily dose of OT will be adapted according to age at the beginning of the study with children aged 2 to < 8 years receiving 8 IU, children aged 8 to 15 years receiving 16 IU and adolescents aged >15 receiving 24 IU. |
| **Number of Patients** | | **Total expected number of patients:** Around 36 patients to be included, to provide a minimum of 36 VFSS for the primary analysis. More patients may be recruited if this is not achieved. |
| **Inclusion Criteria** | | 1. Male or female patients between 2 years and 17 years and 5 months at inclusion. |
| | | 2. Genetically confirmed diagnosis of PWS. |
| | | 3. Parents (or legal representative) have signed the informed consent form and are willing to comply with all study procedures. |
| **Exclusion Criteria** | 1. A history of hypersensitivity to the study drug or drugs with similar chemical structures, to excipients of the product, or to latex; | |
| | 2. Intolerance of intranasal administrations (including when due to a major behavioural problem); | |
| | 3. Hyponatremia; | |
| | 4. Hypokalaemia; | |
| | 5. Prolongation of the QT interval and/or family history of prolongation of the QT interval; | |
| | 6. Concomitant treatment prolonging the QT interval; | |
| | 7. Start of GH treatment within the last 4 weeks before inclusion; | |
| | 8. History of abnormal electrocardiogram (ECG) (validated by a cardiologist); | |
| | 9. Pregnant girls; | |
| | 10. Patient with clinical signs in the context of contact with SARs-COV2 infected person. Patient included in another study protocol on a medicinal product within the last 3 months; | |
| **Efficacy Parameters** | | • VFSS scores and OPOD subscores of the VFSS grid and VFSS-Global impression scales; |
| | | *•* Swallowing symptoms interview; |
| | | *•* Occurrence of feeding events; |
| | | *•* Clinician VFSS global impression scale (severity) |
| **Safety Parameters** | | *•* Adverse events; |
| | | *•* Laboratory safety parameters; |
| | | *•* Vital signs (systolic blood pressure [SBP], diastolic blood pressure [DBP] and heart rate [HR]); |
| | | *•* ECG. |
| **Primary Endpoint** | The percentage of patients showing normalisation of at least 1 of the following abnormal OPOD subscores of the VFSS grid (see Figure 2) after 12 weeks OT / placebo at V2 (groups 1 and 3 vs group 2): | |
| | | *•* Oral and pharyngeal propulsion; |
| | | *•* Initiation and synchronisation of swallowing; |
| | | *•* Oesophageal motility. |
| | As supportive analysis: | |
| | Change from baseline and percentage of patients showing improvement of the VFSS total score in OT vs placebo treated patients at V2 (groups 1 and 3 vs group 2). | |
| | Percentage of patients showing normalisation of at least one of the 3 OPOD subscores at V3 and change from baseline and | |
| | percentage of improvement of the VFSS total score at V3 (OT) vs V2 (placebo) in Group 2. | |
| **Secondary Endpoints** | **Secondary Efficacy Endpoints:** | |
| | The following parameters will be assessed for differences between OT and placebo at V2 or change from baseline (groups 1 and 3 vs group 2): | |
| | | *•* Swallowing symptoms - changes in scores; |
| | | *•* Swallowing symptoms - percentage of improved items; percentage of patients with improved score; |
| | | *•* Occurrence of feeding events - number of events and number of events per patient (overall and choking events); |
| | | *•* Occurrence of feeding events - percentage of patients with events (overall and choking events) |
| | | *•* VFSS global impression scale (severity) - changes in score and percentage of patients with improved score |
| | The percentage of normalised OPOD subscores at V2 which became abnormal at V3, in Group 1. All above parameters presented, as percentage of patients at V3 and as change from baseline to V3 vs change from V2 to V3 in Group 1 (withdrawal effect). | |
| | The percentage of patients showing normalisation of at least 1 of the following OPOD subscores of the VFSS grid in Group 1 vs group 3 at V3 (withdrawal effect): | |
| | | *•* Oral and pharyngeal propulsion; |
| | | *•* Initiation and synchronisation of swallowing; |
| | | *•* Oesophageal motility |
| | The percentage of normalised OPOD subscores at V2 which remains normal at V3, in Group 3; all above parameters presented, as percentage of patients and as change from baseline to V3 vs change from V2 to V3 in Group 3. (Maintenance of efficacy) | |
| | All parameters presented, as percentage of patients and as change from baseline to V4 in all groups (48 weeks for group 3, 36 weeks for group 2 and 24 weeks for group 1) (long-term efficacy). | |
| | **Safety Endpoints:** | |
| | | *•* Adverse events; |
| | | *•* Laboratory safety parameters at V1, V2, V3 and V4; |
| | | *•* Vital signs (SBP, DBP and HR) at V1, V2, V3 and V4; |
| | | • ECG at V1, V2, V3 and V4 |
| **Schedule of Assessments** | Study treatment will be dispensed for a 12-week duration at V1 and V2 and a 24 weeks duration at V3. First administration of | |
| | | dispensed study treatment will be performed the day of the V1, V2- and V3 visit. |
| | | VFSS is to be conducted prior to study treatment administration at all visits. The exact time of VFSS and study treatment administration is to be precisely recorded. Blood samples will be collected prior to study treatment administration at all visits. At V2 and V3, biological sampling will also be performed two times after treatment administration for OT dosage. |
| | | Vital signs and ECG will be recorded at all visits prior to and after study treatment administration. |
| **Duration of Study Per Patient** | | **Maximum total study duration per patient: 48 weeks:** |
| | | Randomised periods: 12 weeks in Part 1, 12 weeks in Part 2 Open Label (Part 3): 24 weeks |

### - STUDY DESIGN

This prospective, multicentre, randomised, double-blind, Phase 3 clinical study is planned to include around 36 PWS patients aged 2-18 years from 5 French centres. The study comprises 3 parts.

The first part is a placebo-controlled, double-blind period to assess the effects of a 12-week course of intranasal OT treatment vs placebo on dysphagia related to OPOD assessed by videofluoroscopy of swallowing and oesophageal transit in male and female children and adolescents with PWS. Groups 1 and 3 will receive OT while Group 2 will receive placebo. Patients will be randomised to 1 of the 3 treatment groups in a 1:1:1 ratio. Randomisation will be stratified by age group (2 to <8 years, 8 to 15 years and >15 years).

In the second part, another double-blind period, Group 1 (patients treated with OT during the first part) will receive 12 weeks of placebo to assess the changes after stopping the treatment whilst Group 2 (patients treated with placebo during the first part) will receive OT and Group 3 (patients also treated with OT during the first part) will stay on OT treatment to assess the maintenance of efficacy.

In the third part, a 24-week open label period of intranasal OT treatment, all patients will receive OT to assess long-term safety and the maintenance of efficacy.

Study treatment will be administered intranasally daily for 48 weeks. The dose of OT will be adapted according to age at the beginning of the study with children aged 2 to < 8 years receiving 8 IU, children aged 8 to 15 years receiving 16 IU and adolescents aged >15 years receiving 24IU.

A telephone conversation will be scheduled with one of the investigators and the parents of PWS patients in the age range. The investigator will answer any question about the objectives, the constraints and predictable risks. After a time for thought, parents who accepted that their child participate in the study will contact the investigator to organize the inclusion in the study.

During the baseline visit (V1) parents will sign the informed consent and eligibility criteria will be verified. Eligible patients will then be included and randomised. A VFSS will be performed with other study procedures and patients will be randomised to the 3 treatment groups prior to the first administration of study treatment.

In case a VFSS has been performed according to routine care and fulfil the requirements regarding quality and acquisition within 4 weeks before the inclusion in the study, the VFSS could be used as a baseline and will not be done at V1.

In the first two parts of the study, study treatment (OT or placebo) will be administered daily for 12 weeks by intranasal route.

The evolution of all parameters will be documented and the first and second treatment period within each group will be compared to assess the effect of OT vs placebo, the effect of treatment withdrawal and the maintenance of efficacy.

The long-term safety profile of intranasal administration of OT will be assessed in the open-label period as well as long-term efficacy.

### - STUDY INTERVENTION

The investigational medicinal product (IMP) in this study is intranasal OT. A matching intranasal placebo solution will also be provided as this is a double-blind study. Both the IMP and the matching placebo will be provided in identical packaging, so as to preserve the study blinding.

The primary characteristics of the IMP and its matching placebo are presented in Table 2 below.

**Table 2 Primary Characteristics of the Investigational Medicinal Product and Placebo**

| | **IMP** | **Placebo** |
|---|---|---|
| INN | Oxytocin | Not applicable |
| Mode of administration | Intranasal | Intranasal |
| Appearance | Clear solution, colourless | Clear solution, colourless |
| Appearance of the container | Amber glass bottle with a spray device with protective cap | Amber glass bottle with a spray device with protective cap |
| Dosage form | Solution | Solution |
| Volume of solution | 6 mL | 6 mL |
| Concentration of solution | 177,76 IU/ mL | Not applicable |
| Weight of a single spray delivered by device | 45 mg | 45 mg |
| Dosage of a single spray delivered by device | 8 IU | Not applicable |

| | | |
|---|---|---|
| IMP = investigational medicinal product; INN = International non-proprietary name; IU = international units. | | |

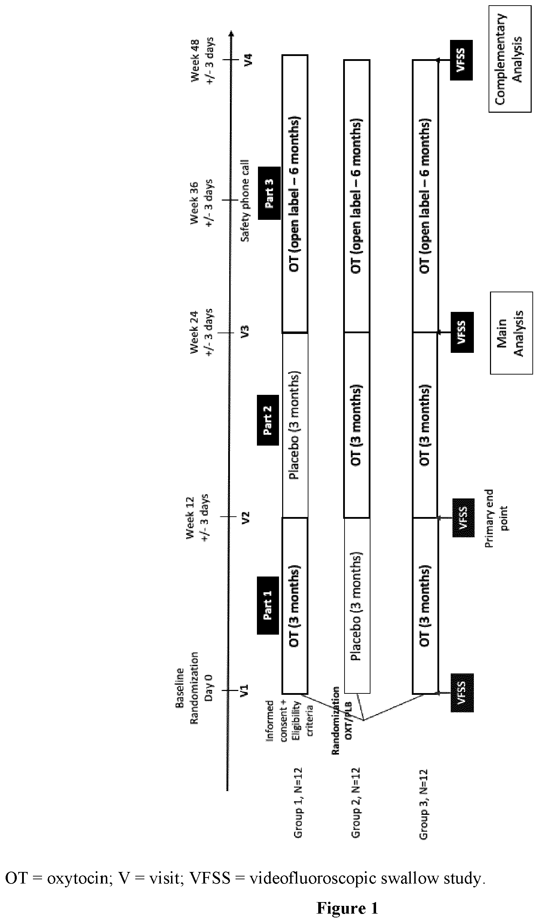

Study treatment will be administered daily for 12 weeks in Part 1 & Part 2 (OT or placebo), and for 24 weeks for the open label period (OT only) by intranasal route (Figure 1).

At V1, the study treatment will be administered by the parent/caregiver or the patient under the supervision of a medical team member. The staff member will also teach the parent(s)/caregiver and/or the patient when and how to deliver the doses, including how to prime the treatment pump and how delayed or missed doses should be handled. Instructions for use on how to position the device for administration will be detailed in a specific study diary to be filled in by the parent/caregiver.

The diary should be completed daily in order to follow treatment compliance and administration time during the Parts 1 and 2. During the open label part, only issues with treatment such as delayed dose, missed dose, or overdose should be recorded at each new OT vial opening/use (every 28 days). Instructions for use and a reminder of instructions on how to handle delayed doses, missed doses and overdose will be provided in the diary. The last administration of each period of study treatment should be performed the day before the visit. At V2, V3 and V4, the parents should bring back the study treatment bottles provided for the previous period of the study as they need to be collected and accounted for by the site staff. At V1, V2 and V3, the parents will be provided with the study treatment for the following part of the study and the first administration will be done at the hospital during the visit.

The dose will be adapted according to age as per the guide presented in Table 3 below.

**Table 3 Dosing Guide**

| **Age range (years)** | **Dose** | **Administration** |
|---|---|---|
| 2 to < 8 | 8 IU | One spray in one nostril (daily) |
| 8 to 15 | 16 IU | One spray in each nostril (daily) |
| >15 | 24 IU | Three sprays in total - Two spays in one nostril and one spray in the other one (daily) |

| | | |
|---|---|---|
| IU = international unit. | | |

## Claims

1. An oxytocin receptor agonist for use in the treatment or prevention of dysphagia in a subject of at least one year old and suffering from a neurodevelopmental disorder.

2. The oxytocin receptor agonist for use according to claim 1 wherein the dysphagia is **characterized by** esophageal dysmotility.

3. The oxytocin receptor agonist for use according to claim 1 or 2 wherein the subject suffers from a hypothalamic dysfunction.

4. The oxytocin receptor agonist for use according to any one of claims 1 to 3 wherein the subject suffers from a genetic neurodevelopmental disorder.

5. The oxytocin receptor agonist for use according to claim 4 wherein the genetic neurodevelopemental disorder is selected from the group consisting of Prader-Willi syndrome and Prader-Willi like syndromes, fragile-X syndrome, DiGeorge/22q11.2 Deletion Syndrome, Down syndrome, Rett syndrome, Noonan syndrome, CHARGE syndrome, Kabuki syndrome, Troyer syndrome, Christianson syndrome, Smith-Magenis syndrome, Alstrom syndrome, syndromic obesity, familial dysautonomia, and Williams syndrome.

6. The oxytocin receptor agonist for use according to claim 1 or 2 wherein the subject suffers from a neurodevelopmental disorder selected from the group consisting of autistic spectrum disorder, cerebral palsy, fetal alcohol spectrum disorder and intellectual disability.

7. The oxytocin receptor agonist for use according to any one of preceding claims wherein the subject is a toddler, a child or a teenager.

8. The oxytocin receptor agonist for use according to any one of preceding claims, wherein dysphagia is **characterized by** at least one of the following clinical signs:
- esophageal stasis,
- esophageal residues,
- abnormal closer of esophageal sphincters, in particular upper esophageal sphincter,
- esophageal distention preferably upper esophageal distention or megaesophagus,
- penetration, inhalation, including silent inhalation
- prolonged or slow esophageal transit,
- decreased peristalsis, and
- esophageal reflux
and wherein said clinical signs are optionally evaluable by videofluoroscopy.

9. The oxytocin receptor agonist for use according to any one of preceding claims, wherein the subject presents esophageal dysmotility **characterized by** one or several of the following features: a slow esophageal transit, a poor esophageal clearance, esophageal reflux, a decreased peristalsis and combinations thereof.

10. The oxytocin receptor agonist for use according to any one of preceding claims, wherein the oxytocin receptor agonist is used to improve or normalize esophageal motility in the subj ect.

11. The oxytocin receptor agonist for use according to any one of preceding claims wherein the subject has experimented, experiments or is at risk of experimenting one or several of the following disorders: esophageal food bolus obstruction, pulmonary aspiration, recurrent pulmonary infection, aspiration pneumonia, choking, regurgitations, nasal regurgitation and meryscism.

12. The oxytocin receptor agonist for use according to any one of preceding claims, wherein said oxytocin receptor agonist is selected from the group consisting of oxytocin, carbetocin, [Thr⁴]OT, HO[Thr⁴]OT, [Thr⁴, Gly⁷]OT, HO[Thr⁴, Gly⁷]OT, Lipo-oxytocin-1 (LOT-1), demoxytocin, merotocin, demoxytocin, lipo-oxytocin-1 (LOT-1), TC OT 39, and WAY-267464. LIT-001.

13. The oxytocin receptor agonist for use according to any one of preceding claims which is oxytocin or a pharmaceutically acceptable salt thereof.

14. The oxytocin receptor agonist for use according to claim 13, wherein the neurodevelopmental disorder is Prader-Willi syndrome or a Prader-Willi like disorder.

15. The oxytocin receptor agonist for use according to any one of claims 13-14, wherein the subject suffers from silent dysphagia.

16. The oxytocin receptor agonist for use according to any one of claims 13-15, which is administered by intranasal route, preferably at a daily dosage from 2 IU to 48 IU.

## Patentansprüche

1. Oxytocin-Rezeptor-Agonist zur Verwendung bei der Behandlung oder Vorbeugung von Dysphagie bei einem Patienten von mindestens einem Jahr und der an einer neurologischen Entwicklungsstörung leidet.

2. Der Oxytocin-Rezeptor-Agonist zur Verwendung nach Anspruch 1, wobei die Dysphagie durch eine Motilitätsstörung der Speiseröhre gekennzeichnet ist.

3. Der Oxytocin-Rezeptor-Agonist zur Verwendung nach Anspruch 1 oder 2, wobei das Subjekt an einer hypothalamischen Dysfunktion leidet.

4. Der Oxytocin-Rezeptor-Agonist zur Verwendung nach einem der Ansprüche 1 bis 3, wobei das Subjekt an einer genetischen neurologischen Entwicklungsstörung leidet.

5. Der Oxytocin-Rezeptor-Agonist zur Verwendung nach Anspruch 4, wobei die genetische neurologische Entwicklungsstörung ausgewählt ist aus der Gruppe bestehend aus Prader-Willi-Syndrom und Prader-Willi-ähnlichen Syndromen, Fragile-X-Syndrom, DiGeorge/22ql1.2-Deletionssyndrom, Down-Syndrom, Rett-Syndrom, Noonan-Syndrom, CHARGE-Syndrom, Kabuki-Syndrom, Troyer-Syndrom, Christianson-Syndrom, Smith-Magenis-Syndrom, Alstrom-Syndrom, syndromische Fettleibigkeit (syndromic obesity), familiäre Dysautonomie und Williams-Syndrom.

6. Der Oxytocin-Rezeptor-Agonist zur Verwendung nach Anspruch 1 oder 2, wobei das Subjekt an einer neurologischen Entwicklungsstörung leidet, ausgewählt aus der Gruppe bestehend aus Autismus-Spektrum-Störung, Zerebralparese, fetaler Alkohol-Spektrum-Störung und geistiger Behinderung.

7. Der Oxytocin-Rezeptor-Agonist zur Verwendung nach einem der vorhergehenden Ansprüche, wobei es sich bei dem Subjekt um ein Kleinkind, ein Kind oder einen Teenager handelt.

8. Oxytocin-Rezeptor-Agonist zur Verwendung nach einem der vorhergehenden Ansprüche, wobei Dysphagie durch mindestens eines der folgenden klinischen Anzeichen gekennzeichnet ist:
- Ösophagusstauung,
- Speiseröhrenrückstände,
- abnormaler Schluss der Schließmuskeln der Speiseröhre, insbesondere des oberen Schließmuskels der Speiseröhre,
- Ösophagusdehnung, vorzugsweise obere Ösophagusdehnung oder Megaösophagus,
- Penetration, Inhalation, einschließlich stiller Inhalation
- verlängerter oder langsamer Ösophagustransit,
- verminderte Peristaltik und
- Reflux der Speiseröhre
und wobei die klinischen Anzeichen optional durch Videofluoroskopie auswertbar sind.

9. Der Oxytocin-Rezeptor-Agonist zur Verwendung nach einem der vorhergehenden Ansprüche, wobei das Subjekt eine ösophageale Dysmotilität aufweist, die durch eines oder mehrere der folgenden Merkmale gekennzeichnet ist: ein langsamer Ösophagustransit, eine schlechte ösophageale Clearance, ösophagealer Reflux, eine verminderte Peristaltik und Kombinationen davon.

10. Der Oxytocin-Rezeptor-Agonist zur Verwendung nach einem der vorhergehenden Ansprüche, wobei der Oxytocin-Rezeptor-Agonist zur Verbesserung oder Normalisierung der Motilität der Speiseröhre bei dem Patienten verwendet wird.

11. Der Oxytocin-Rezeptor-Agonist zur Verwendung nach einem der vorhergehenden Ansprüche, wobei das Subjekt eine oder mehrere der folgenden Störungen erlitten hat, erleidet oder Gefahr läuft, sie zu erleiden: Speiseröhrenobstruktion durch Nahrungsbolus, Lungenaspiration, rezidivierende Lungeninfektion, Aspirationspneumonie, Ersticken, Aufstoßen, Naseninsuffizienz und Wiederkäuen ("meryscism").

12. Der Oxytocin-Rezeptor-Agonist zur Verwendung nach einem der vorhergehenden Ansprüche, wobei der Oxytocin-Rezeptor-Agonist ausgewählt ist aus der Gruppe bestehend aus Oxytocin, Carbetocin, [Thr⁴]OT, HO[Thr⁴]OT, [Thr⁴, Gly⁷]OT, HO[Thr⁴, Gly⁷]OT, Lipo-Oxytocin-1 (LOT-1), Demoxytocin, Merotocin, Demoxytocin, Lipooxytocin-1 (LOT-1), TC OT 39 und WAY-267464. LIT-001.

13. Der Oxytocin-Rezeptor-Agonist zur Verwendung nach einem der vorhergehenden Ansprüche, bei dem es sich um Oxytocin oder ein pharmazeutisch verträgliches Salz davon handelt.

14. Der Oxytocin-Rezeptor-Agonist zur Verwendung nach Anspruch 13, wobei die neurologische Entwicklungsstörung das Prader-Willi-Syndrom oder eine Prader-Williähnliche Störung ist.

15. Der Oxytocin-Rezeptor-Agonist zur Verwendung nach einem der Ansprüche 13-14, wobei das Subjekt an stiller Dysphagie leidet.

16. Der Oxytocin-Rezeptor-Agonist zur Verwendung nach einem der Ansprüche 13-15, der auf intranasalem Weg verabreicht wird, vorzugsweise in einer täglichen Dosierung von 2 IU bis 48 IU.

## Revendications

1. Agoniste d'un récepteur de l'ocytocine pour une utilisation dans le traitement ou la prévention d'une dysphagie chez un sujet âgé d'au moins un an et souffrant d'un trouble neurodéveloppemental.

2. Agoniste d'un récepteur de l'ocytocine pour une utilisation selon la revendication 1, dans lequel la dysphagie est **caractérisée par** une dysmotilité oesophagienne.

3. Agoniste d'un récepteur de l'ocytocine pour une utilisation selon la revendication 1 ou 2, dans lequel le sujet souffre d'un dysfonctionnement hypothalamique.

4. Agoniste d'un récepteur de l'ocytocine pour une utilisation selon l'une quelconque des revendications 1 à 3, dans lequel le sujet souffre d'un trouble neurodéveloppemental génétique.

5. Agoniste d'un récepteur de l'ocytocine pour une utilisation selon la revendication 4, dans lequel le trouble neurodéveloppemental génétique est choisi dans le groupe constitué par le syndrome de Prader-Willi et les syndromes de type Prader-Willi, le syndrome de l'X fragile, le syndrome de délétion de DiGeorge/22q11.2, le syndrome de Down, le syndrome de Rett, le syndrome de Noonan, le syndrome CHARGE, le syndrome de Kabuki, le syndrome de Troyer, le syndrome de Christianson, le syndrome de Smith-Magenis, le syndrome d'Alstrom, l'obésité syndromique, la dysautonomie familiale, et le syndrome de Williams.

6. Agoniste d'un récepteur de l'ocytocine pour une utilisation selon la revendication 1 ou 2, dans lequel le sujet souffre d'un trouble neurodéveloppemental choisi dans le groupe constitué par un trouble du spectre autistique, une paralysie cérébrale, un trouble du spectre d'alcoolisation foetale et une déficience intellectuelle.

7. Agoniste d'un récepteur de l'ocytocine pour une utilisation selon l'une quelconque des revendications précédentes, dans lequel le sujet est un enfant en bas-âge, un enfant ou un adolescent.

8. Agoniste d'un récepteur de l'ocytocine pour une utilisation selon l'une quelconque des revendications précédentes, dans lequel la dysphagie est **caractérisée par** au moins un des signes cliniques suivants :
- une stase oesophagienne,
- des résidus oesophagiens,
- une fermeture anormale des sphincters oesophagiens, en particulier du sphincter oesophagien supérieur,
- une distension oesophagienne, de préférence une distension oesophagienne supérieure ou un mégaoesophage,
- une pénétration, une inhalation, y compris une inhalation silencieuse,
- un transit oesophagien prolongé ou lent,
- un péristaltisme réduit, et
- un reflux oesophagien,
et dans lequel lesdits signes cliniques sont éventuellement évaluables par vidéo-fluoroscopie.

9. Agoniste d'un récepteur de l'ocytocine pour une utilisation selon l'une quelconque des revendications précédentes, dans lequel le sujet présente une dysmotilité oesophagienne **caractérisée par** une ou plusieurs des caractéristiques suivantes : un transit oesophagien lent, une clairance oesophagienne faible, un reflux oesophagien, un péristaltisme réduit et leurs combinaisons.

10. Agoniste d'un récepteur de l'ocytocine pour une utilisation selon l'une quelconque des revendications précédentes, lequel agoniste d'un récepteur de l'ocytocine est utilisé pour améliorer ou normaliser la motilité oesophagienne chez le sujet.

11. Agoniste d'un récepteur de l'ocytocine pour une utilisation selon l'une quelconque des revendications précédentes, dans lequel le sujet a subi, subit, ou est à risque de subir, un ou plusieurs des troubles suivants : une obstruction oesophagienne par le bol alimentaire, une aspiration pulmonaire, une infection pulmonaire récurrente, une pneumonie par aspiration, un étouffement, des régurgitations, une régurgitation nasale et un mérycisme.

12. Agoniste d'un récepteur de l'ocytocine pour une utilisation selon l'une quelconque des revendications précédentes, lequel agoniste d'un récepteur de l'ocytocine est choisi dans le groupe constitué par l'ocytocine, la carbétocine, [Thr⁴]OT, HO[Thr⁴]OT, [Thr⁴, Gly⁷]OT, HO[Thr⁴, Gly⁷]OT, la lipo-ocytocine-1 (LOT-1), la démocytocine, la mérotocine, la démocytocine, la lipo-ocytocine-1 (LOT-1), TC OT 39, et WAY-267464, LIT-001.

13. Agoniste d'un récepteur de l'ocytocine pour une utilisation selon l'une quelconque des revendications précédentes, ledit agoniste étant l'ocytocine ou un sel pharmaceutiquement acceptable de celle-ci.

14. Agoniste d'un récepteur de l'ocytocine pour une utilisation selon la revendication 13, dans lequel le trouble neurodéveloppemental est le syndrome de Prader-Willi ou un syndrome de type Prader-Willi.

15. Agoniste d'un récepteur de l'ocytocine pour une utilisation selon l'une quelconque des revendications 13 et 14, dans lequel le sujet souffre de dysphagie silencieuse.

16. Agoniste d'un récepteur de l'ocytocine pour une utilisation selon l'une quelconque des revendications 13 à 15, qui est administré par voie intranasale, de préférence à une dose quotidienne de 2 UI à 48 UI.
